(19) Europäisches Patentamt
European Patent Office
Office européen des brevets

(11) **EP 1 985 633 A1**

(12) **EUROPEAN PATENT APPLICATION**

(43) Date of publication:
29.10.2008 Bulletin 2008/44

(51) Int Cl.:
*C07K 16/28* (2006.01)  *A61K 35/14* (2006.01)
*A61K 39/395* (2006.01)  *A61P 35/00* (2006.01)

(21) Application number: 07290522.7

(22) Date of filing: 26.04.2007

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK RS

(83) Declaration under Rule 28(4) EPC (expert solution)

(71) Applicants:
• **LFB Biotechnologies**
91940 Les Ulis (FR)
• **I.D.M. IMMUNO-DESIGNED MOLECULES**
75011 Paris (FR)

(72) Inventors:
• **Fernandez, Nadine**
92130 Issy-les-Moulineaux (FR)
• **de Romeuf, Christophe**
59120 Lambersart (FR)
• **Urbain, Rémi**
94240 L'Hay les Roses (FR)
• **Bartholeyns, Jacques**
49730 Turquant (FR)
• **Boyer, Aurélie**
94170 Le Perreux sur Marne (FR)

(74) Representative: **Hirsch & Associés**
58, avenue Marceau
75008 Paris (FR)

(54) **Kit of parts for the treatment of cancer or infectious diseases**

(57) The application relates to a kit of parts for the treatment of cancer or infectious diseases, comprising activated macrophages and a monoclonal antibody, wherein the affinity of the Fc region of said antibody to Fc gamma receptor III (CD16) is higher than the affinity of IgG polyclonal antibodies to said Fc gamma receptor III.

**Description**

**TECHNICAL FIELD**

The invention relates generally to the treatment of hematopoietic tumor or autoimmune diseases, in particular through the use of activated macrophages and a monoclonal antibody.

**BACKGROUND OF THE INVENTION**

The interaction of antibody-antigen complexes with cells of the immune system results in a wide array of responses, ranging from effector functions such as antibody-dependent cell cytotoxicity (ADCC), mast cell degranulation, and phagocytosis to immunomodulatory signals such as regulating lymphocyte proliferation and antibody secretion. All these interactions are initiated through the binding of the Fc domain of antibodies or immune complexes to specialized cell surface receptors on hematopoietic cells.

The Fc receptors are surface glycoproteins that can bind the Fc portion of immunoglobulin (Ig) molecules. Fc receptors are defined by their specificity for immunoglobulin subtypes. Fc receptors for IgG are referred to as FcγR, for IgE as FcεR, and for IgA as FcαR. Different accessory cells bear Fc receptors for antibodies of different isotype, and the isotype of the antibody determines which accessory cells will be engaged in a given response (reviewed by Gerber J. S. et al. 2001 Microbes and Infection, 3: 131-139; Billadeau D. D. et al. 2002, The Journal of Clinical Investigation, 2 (109): 161-1681; Ravetch J. V. et al. , 2001 Annu. Rev. Immunol. 19:275-90). There are three known FcγRs, designated FcγRI(CD64), FcγRII(CD32), and FcγRIII(CD16).

In autoimmune and/or inflammatory disorders, the immune system triggers an inflammatory response when there are no foreign substances to fight and the body's normally protective immune system causes damage to its own tissues by mistakenly attacking self. There are many different autoimmune disorders which affect the body in different ways. For example, the brain is affected in individuals with multiple sclerosis, the gut is affected in individuals with Crohn's disease, and the synovium, bone and cartilage of various joints are affected in individuals with rheumatoid arthritis. As autoimmune disorders progress, destruction of one or more types of body tissues, abnormal growth of an organ, or changes in organ function may result. The autoimmune disorder may affect only one organ or tissue type or may affect multiple organs and tissues. Organs and tissues commonly affected by autoimmune disorders include hematopoietic cells, blood vessels, connective tissues, endocrine glands (e.g., the thyroid or pancreas), muscles, joints, and skin. Autoimmune diseases are often associated with an inflammatory disease. Inflammatory disease such as rheumatoid arthritis (RA) and juvenile rheumatoid arthritis are types of inflammatory arthritis. Arthritis is a general term that describes inflammation in joints. Besides rheumatoid arthritis, other types of arthritis associated with inflammation include the following: psoriatic arthritis, Reiter's syndrome, ankylosing spondylitis arthritis, and gouty arthritis.

Many current treatments have a high incidence of side effects or cannot completely prevent disease progression. So far, no treatment is ideal, and there is no cure. Novel therapeutics are needed that more effectively treat inflammatory and/or autoimmune disorders.

A tumor is a neoplastic mass resulting from abnormal uncontrolled cell growth which can be benign or malignant. Benign tumors generally remain localized. Malignant tumors are collectively termed cancers. The term "malignant" generally means that the tumor can invade and destroy neighboring body structures and spread to distant sites to cause death (for review, see Robbins and Angell, 1976, Basic Pathology, 2d Ed., W.B. Saunders Co., Philadelphia, pp. 68-122). Cancer can arise in many sites of the body and behave differently depending upon its origin. Cancerous cells destroy the part of the body in which they originate and then spread to other part(s) of the body where they start new growth and cause more destruction.

Currently, cancer therapy may involve surgery, chemotherapy, hormonal therapy and/or radiation treatment to eradicate neoplastic cells in a patient (Stockdale, 1998, "Principles of Cancer Patient Management", in Scientific American: Medicine, vol. 3, Rubenstein and Federman, eds., Chapter 12, Section IV). Despite the availability of a variety of chemotherapeutic agents, chemotherapy has many drawbacks. Almost all chemotherapeutic agents are toxic, and chemotherapy causes significant, and often dangerous, side effects, including severe nausea, bone marrow depression, immunosuppression, etc.

In conventional therapy, residual tumor cells are left undamaged due to chemoresistance or due to the fact that these cells are shaded in protected areas or located in hypoxic areas poorly vascularized and not accessible to conventional treatments. The genetic instability and heterogeneity of tumors indeed allow them to adapt and to develop resistance to therapies.

The beneficial effects of chemotherapy can be compromised by cellular mechanisms that allow neoplastic tissue to evade the toxicity of drugs. In some cases, pleiotropic resistance to a variety of unrelated drugs has been observed, and this phenomenon has been called multidrug resistance. Development of multidrug resistance, is frequently observed in second or third intention cytotoxic treatment of cancer. Resistance to chemotherapy, whether it is intrinsic or acquired, is a major cause of failure in the curative treatment of neoplastic malignances. There is a significant need for alternative cancer treatments, particularly for treatment of cancer that has proved refractory to standard cancer treatments, such as surgery, radiation therapy, chemotherapy, and hormonal therapy.

A promising alternative is immunotherapy, in which cancer cells are specifically targeted by cancer antigen-specific antibodies. Major efforts have been directed at harnessing the specificity of the immune response, for example, hybridoma technology has enabled the development of tumor selective monoclonal antibodies (See Green M. C. et al., 2000, Cancer Treat Rev., 26: 269-286; Weiner L M, 1999, Semin Oncol. 26(suppl. 14):43-51). These molecules of high specificity and stability have become key resources in the therapeutic, diagnostic and drug discovery fields to treat various immunological disorders and malignancies of different organs.

Many technical efforts have been made to generate a second-generation mAb with decreased immunogenicity and with optimised effector functions.

B cells are the immune system's "arms factories," producing antibodies that target invading microbes for destruction. Abnormal B cell proliferation causes such leukemias as multiple myeloma and acute lymphoblastic leukemia. They also have a critical role in antibody mediated autoimmune diseases such as rheumatoid arthritis and lupus. Preliminary clinical studies suggest therapeutic benefits in patients with classic autoantibody-mediated syndromes, such as autoimmune cytopenias. B-cell-targeted therapy promises to rapidly secure an important place in the treatment of autoantibody-associated conditions.

Rituximab (marketed as Rituxan® by Genentech and MabThera® by Roche) was the first FDA-approved monoclonal antibody and was developed at IDEC Pharmaceuticals (see U.S. Pat. Nos. 5,843,439; 5,776,456 and 5,736, 137) for treatment of human B-cell lymphoma, an haematological cancer (Reff et al., Blood, 83: 435-445 (1994)). Rituxan is a chimeric, anti-CD20 monoclonal antibody (mab). CD20 is a tetraspanning transmembrane phospho-protein that is expressed predominantly in pre-B cells and in mature peripheral B cells in humans and mice. In humans, CD20 is also strongly and homogeneously expressed on most mature B-cell malignancies, except for chronic lymphocytic leukemia (CLL) cells, where expression is more variable. Rituxan® has been first approved for the treatment of relapsed or refractory, CD20+, B-cell low-grade Non-Hodgkin's Lymphoma (NHL). In 2006, it has been approved in combination with chemotherapy for three others types of NHL. Rituxan is also approved in combination with methotrexate in adult patients with moderately-to-severely active rheumatoid arthritis who have had an inadequate response to one or more tumor necrosis factor antagonist therapies.

Other haematological malignant disorders treated with Rituxan® include follicular center cell lymphoma (FCC), mantle cell lymphoma (MCL), diffuse large cell lymphoma (DLCL), small lymphocytic lymphoma/chronic lymphocytic leukemia (SLL/CLL) and auto-immune diseases such as systemic lupus or idiopathic thrombocytopenic purpura. Despite its clinical success, the mechanism of antitumor activity of Rituxan® *in vivo* is still a matter of debate. There is extensive evidence for the importance of complement-dependent cytotoxicity in anti-CD20-induced lymphoma cell killing (Kennedy AD, Beum PV, Solga MD, et al. Rituximab infusion promotes rapid complement depletion and acute CD20 loss in chronic lymphocytic leukaemia. J. Immunol. 2004; 172:3280-3288). In chronic lymphocytic leukemia (CLL), clinical results with Rituximab as a single agent have been less encouraging than in other B cell lymphoma (Hilmen P. Advancing therapy for chronic lymphocytic leukaemia- the role of rituximab. Semin Oncol. 2004; 31:22-26). Possible explanations for this lack of efficacy are the relatively low CD20 density on CLL cells compared to normal B cell or lymphoma cell lines and the poor capacity of Rituximab to induce ADCC (Antibody Dependant Cellular Cytotoxicity) response against primary B-CLL cells *in vitro.*

Innovative therapies capable of inducing the lysis of tumor cells including those expressing low level of antigen are then needed.

Macrophages play a major role in the antitumoral response, and they are able to be activated by immunological activators against cancer cells (Adams D. and Hamilton T.: Activation of macrophages for tumor cell kill: effector mechanism and regulation. In Heppner & Fulton (eds), Macrophages and cancer. CRC Press, 1988, p. 27; Fidler I.: Macrophages and metastases. A biological approach to cancer therapy. Cancer Res. 45: 4714, 1985).

Furthermore, macrophages, or other cells derived from monocytes or from their precursors, with their strong capacity for endocytosis, digestion, and surface antigen presentation, are capable of inducing a specific immune response.

Macrophages presenting cytotoxic activity which is particularly significant (also designated as activated macrophages or MAK (registered trade mark)) as well as a culture medium and process for obtaining said macrophages were the subject matter of international patent application WO 94/26875, filed on May 18, 1993, and of patent EP 0 806 959.

In order to overcome the poor efficiency of rituximab in CLL, its combination with ex *vivo* activated macrophages has been evaluated for killing primary CLL cells (Lefebvre ML, Stefan W. Krause, Salcedo M, Nardin A. Ex vivo activated human macrophages kill chronic lymphocytic leukemia cells in the presence of Rituximab: mechanism of antibody-dependent cellular cytotoxicity and impact of human serum. J. Immunother; vol. 29, nr 4: 388-397, 2006). In presence of the rituximab, IFN-γ activated macrophages can kill primary CLL cells at Effector:Target (E:T) ratios as low as 0.4:1. However, in accordance with previous studies, the addition of human serum significantly inhibited the ADCC via immunoglobulins G (IgG) competition on Fc receptors. The use of high rituximab concentration together with high E:T ratios permitted in some cases to partially overcome the serum inhibition.

Therefore, in spite of the existence of encouraging tools for the treatment of cancers and of infectious therapies, there still exists a strong need for new immunotherapeutic products, displaying greater efficiency and safety than the existing products.

## SUMMARY OF THE INVENTION

Accordingly, it is an object of the present invention to provide a kit of parts for treating haematopoietic tumors, including abnormal B-cell proliferation such as chronic lymphocytic leukaemia (CLL), or autoimmune disease, comprising an activated macrophage and a monoclonal antibody directed against an antigen expressed by tumor cells or involved in auto-immune diseases.

The present invention concerns the combined use of activated macrophage and antibody in a subject suffering from a hematopoietic tumor or autoimmune disease. More specifically, the present invention provides a kit of parts for the treatment of hematopoietic tumor or autoimmune disease comprising an activated macrophage and a monoclonal antibody wherein the affinity of the Fc region of said antibody to Fc gamma receptor is higher than the affinity of IgG polyclonal antibodies to said Fc gamma receptor.

Furthermore, the invention concerns a use of such kit of parts as a medicament for the treatment of a patient suffering from a blood cancer or autoimmune disease.

A first object of the invention is a kit of parts for the treatment of cancer or infectious diseases, preferably hematologic cancer or auto-immune disease, comprising activated macrophages and a monoclonal antibody, wherein the affinity of the Fc region of said antibody to Fcγreceptor III (CD16) is higher than the affinity of IgG polyclonal antibodies to said CD16. Advantageously, the said affinity of the Fc region of said antibody is at least of $2.10^6$ $M^{-1}$.

Advantageously, as a result of their high affinity of the Fc region of said antibody to CD16, said monoclonal antibody is not displaced by IgG polyclonal antibodies, especially by IgG present in blood serum.

Advantageously, said monoclonal antibody binds to CD16 of said macrophage with an affinity of at least $2.10^6$ $M^{-1}$, and more advantageously of at least $1.10^7$ $M^{-1}$, or preferably of $1.10^8$ $M^{-1}$ or even of $1.10^9$ $M^{-1}$, as determined by Scatchard analysis or BIAcore technology (Label-free surface plasmon resonance based technology).

Advantageously, the monoclonal antibody is produced in the form of a composition of monoclonal antibodies, each of said monoclonal antibody having N-glycoside-linked sugar chains bound on the Fcγ glycosylation site (Asn 297, EU numbering), and wherein among said N-glycoside-linked sugar chains of all the antibodies of the composition, the fucose content is less than 65%.

In a preferred embodiment, said activated macrophage is a MAK® (IDM, Paris, France).

In another preferred embodiment, said antibody has a variable region directed against an antigen expressed by tumor cells or involved in auto-immune diseases.

Advantageously, said antigen is CD20.

In a preferred embodiment, said monoclonal antibody is EMAB6, produced by the clone R509, deposited to the CNCM under the accession number I-3314.

Advantageously, said monoclonal antibody is EMAB603, produced by the clone R603, deposited to the CNCM under the accession number I-3529.

Optionally, said cancer is chronic lymphoid leukaemia (CLL).

Another object of the invention is the use of a kit of parts for the preparation of a medicament.

Another object of the invention is the use of the kit of parts for the preparation of a medicament for the treatment of hematopoietic tumor or autoimmune disease.

Advantageously, the hematopoietic tumor comprises leukemia, lymphoma and myeloma. More particularly, the hematopoietic tumor comprising B-cell malignancy is selected from the group consisting of indolent forms of B-cell lymphomas, aggressive forms of B-cell lymphomas, chronic lymphatic leukemias, acute lymphatic leukemias, Waldenstrom's macroglobulinemia and multiple myeloma.

Advantageously, the autoimmune disease comprises immune dysregulation disease, inflammatory disorders, organ graft rejection or graft versus host disease, septicemia and septic shock, Hashimoto's thyroiditis, pernicious anemia, sarcoidosis, primary biliary cirrhosis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, rapidly progressive glomerulonephritis, psoriasis, fibrosing alveolitis, ankylosing spondylitis, Goodpasture's syndrome, thromboangitis obliterans, ulcerative colitis, erythema multiforme, IgA nephropathy, polyarteritis nodosa, Henoch-Schonlein purpura, post-streptococcal nephritis, erythema nodosum, Takayasu's arteritis, Addison's disease, diabetes mellitus, rheumatoid arthritis, systemic lupus erythematosus, Sjogren's syndrome, Sydenham's chorea, lupus nephritis, rheumatic fever, polyglandular syndromes, bullous pemphigoid, multiple sclerosis, autoimmune inner ear disease, myasthenia gravis, Reiter's syndrome, Graves disease, auto-immune cytopenia and other adult and children haematological complications such as auto-immune anemia, cold agglutinin disease, acute and chronic idiopathic thrombopenic purpura, acquired auto-immune hemophilia, children systemic auto-immune diseases including the antiphospholipide syndrome, chronic juvenile arthritis or inflammatory myositis.

**DETAILED DESCRIPTION**

Kit of parts

The term « kit of parts » as used herein designates a combined preparation containing, as active substance, activated macrophages and a monoclonal antibody, for the simultaneous, separate or sequential use, for the treatment of hematologic diseases or autoimmune diseases. Preferably macrophages and antibodies are associated to form a single active unit before injection to the patient.

Those two individual components (also designated as "the two active ingredients") form a functional unit, i.e. a functional true combination through a purpose-directed application. Due to their use in the kit of parts of the invention, the two active ingredients (activated macrophages and monoclonal antibody) show a joint effect.

Activated macrophages

The term « activated macrophages » as used herein is intended to include macrophages which have at least one of the following properties:

- their cytotoxic activity with IFN-γ is increased by about 20 to about 40 % with respect to standard macrophages, and is preferably of about 93 %;
- the extension of deactivation of cytotoxic activity in reply to an activation of IFN-γ is in a ratio such that after 60h of activation with IFN-γ, cytotoxic activity is higher than or equal to 30 %, preferably of about 55 %, compared to the maximum cytotoxic activity displayed by the macrophages due to IFN-γ activation, with said cytotoxic activity being measured as the percentage of inhibition of 3-H thymidine (tritiated thymidine) incorporation by target tumoral cells, particularly U 937 cells.

The expression "standard macrophages" corresponds to those obtained by the culture of monocytes in a standard medium such as RPMI 1640, AIMV or Iscove modified media.

"Cytotoxic activity" corresponds to that measured as a percentage of inhibition of 3-H thymidine incorporation by target tumoral cells, particularly U937 cells (human histiocytic cell line, ATCC CRL 1593.2), and a test for this measure is hereafter given: differentiated macrophages were seeded into 96-well flat microtiter plates at 104 macrophages/well in 0.2 ml. After 2h incubation at 37°C in 5 % CO2 humidified atmosphere, the medium was removed and replaced by IMDM + 5 % human AB serum optionally containing 250 U/ml of recombinant human IFN-γ; plates were further incubated overnight. After washing, $10^4$ U937 tumor cells in 0.2 ml fresh medium (IMDM + 10 % FCS) were added to each well. After 24h contact, 0.1 /Xi of tritiated thymidine was added to each well for a further 24h incubation.

The cells were collected and the determination of incorporated radioactivity on fiber glass filters was carried out by 0 counting. Percentage inhibition of thymidine incorporation by tumor cells could be calculated according to the formula:

$$\frac{(\text{cpmT} - \text{cpmTM}) \times 100}{\text{cpmT}}$$

where cpmT = radioactivity in control tumor cells ;
cpmTM = radioactivity in tumor cells + macrophage mixed culture.

It is to be noted that such a method enables the determination of the three main types of antitumoral activity of the macrophages described in the invention, i.e.: cytolysis, cytostasis and phagocytosis.

The macrophages originate from human subjects, who can be either healthy subjects in case of allogeneic therapy or more preferably from the patient himself, suffering from various diseases.

Preferably, the macrophages originate from the patient to be treated by adoptive immunotherapy.

Activated macrophages as well as a culture medium and process for obtaining said macrophages are detailed in the international patent application WO 94/26875 or in the European patent EP 0806959, which are included herein for references.

The process for preparing macrophages defined in the above mentioned patent application WO 94/26875 is carried out with a composition rich in blood cells obtained by apheresis from a healthy individual or from a patient, and includes a stage of monocyte culture in a culture medium containing GM-CSF (Granulocyte-Macrophage colony stimulating factor) and 2% of autologous serum. Advantageously, the monocytes are cultivated in the presence of lymphocytes for about 6 to 7 days. Then, the differentiated macrophages thus obtained are activated in the culture medium by the addition of IFN-γ. On day 7, the macrophages can be enriched by elutriation.

The culture step may be preceded by a separation step of mononuclear cells on the one hand and red cells, granulocytes and part of the platelets contained in the composition derived from blood obtained by apheresis on the other hand ; and then by a purification step, by washing part of the blood platelets and anticoagulants remaining after the preceding separation step.

For example, the activated macrophage may be prepared according to the method comprising the following steps :

1) recovery of blood derived mononuclear cells directly from blood apheresis or from blood bag collection, followed by centrifugation, to eliminate a substantial part of red blood cells, granulocytes and platelets, and collection of peripheral blood leukocytes ;

2) washing peripheral blood leukocytes obtained from the preceeding steps for instance by centrifugation (to remove 90% of platelets, red blood cells and debris) to obtain mononuclear cells ;

3) resuspension of the total mononuclear cells (monocytes + lymphocytes) obtained from the preceeding step in culture medium (RPMI or IMDM type) at $10^6$ to $2.10^7$ cells/ml, and culture for 5 to 10 days at 37°C under $O_2/CO_2$ atmosphere in hydrophobic gas permeable bags, to obtain monocyte derived cells and contaminating lymphocytes ;

4) a purification step, in particular an elutriation step in which the macrophages activated are separated from the other constituents of the composition obtained after the culture step.

In a preferred embodiment of the present invention, said activated macrophage is a macrophage activated killer, MAK® (IDM, Paris, France), described in the international patent application WO 94/26875 or in the European patent EP 0806959 or in the document Immunobiology, 210, (2005), 267-277: Anti-tumor properties of human-activated macrophages produced in large scale for clinical application ; Veronique Baron-Bodo, Paula Doceur, Marie-Laure Lefebvre, Karine Labroquere, Catherine Defaye, Christophe Cambouris, Didier Prigent, Margarita Salcedo, Aurélie Boyer, Alessandra Nardin, which are incorporated herein by reference.

Monoclonal antibody

The term "antibody" or "immunoglobulin" (used interchangeably herein) refers to an antigen-binding protein having a basic four-polypeptide chain structure consisting of two heavy and two light chains, said chains being stabilized, for example, by interchain disulfide bonds, which has the ability to specifically bind antigen. Each chain is divided into regions or domains consisting of around 110 amino acid residues. The light chain has two domains and the heavy has four. The N-terminal domain at the tip of the arms of the "Y" on both the heavy and light chain are known to be variable in amino acid sequence composition and are thus called "variable region" (VL and VH). The other domains are called constant for a similar reason (CL, CH1, CH2, CH3).

The variable domains show three regions of hypervariability in sequence called the complementarity determining regions (CDRs). They differ in length and sequence between different antibodies and are mainly responsible for the specificity (recognition) and affinity (binding) of the antibodies to their target markers. Proteolytic digestion of antibodies releases different fragments termed Fv (Fragment variable), Fab (Fragment antigen binding) and Fc (Fragment crystallization). Note that antibody engineering can join the separate segments of the heavy and light chains in the Fv with a flexible peptide linker to form a single-chain Fv (scFv).

The Fc region mediates the effector function of the antibodies, which are induced as a result of interaction of the antibody with its antigen via the variable moiety. The antibody of the present invention comprises a variable region and a constant region (Fc).

In a preferred embodiment of the invention, the constant region (Fc) of the monoclonal antibody is human. Human constant region DNA sequences can be isolated in accordance with well known procedures from a variety of human cells, for example immortalized B cells (see Kabat et al., supra, and Liu et al., WO 87/02671) (each of which is incorporated by reference in its entirety for all purposes). Ordinarily, the antibody will contain both light chain and heavy chain constant regions. The heavy chain constant region usually includes CH1, hinge, CH2, CH3, and CH4 regions. The antibodies described herein include antibodies having all types of constant regions, including IgM, IgG, IgD, IgA and IgE, and any isotype, including IgG1, IgG2, IgG3 and IgG4. The choice of constant region depends, in part, whether antibody-dependent complement and/or cellular mediated toxicity is desired. For example, isotopes IgG1 and IgG3 have complement activity and isotypes IgG2 and IgG4 do not.

The term "monoclonal antibody" is intended to include a preparation of antibody whose specificity is unique and identical. These antibodies have an identical amino-acid sequence because they are produced by one type of cell line and are all clones of a single parent cell. It is to be noted that post-translational modifications, like glycosylation, depend on the nature of the cell line, and that as such the monoclonal antibodies may not be identical with respect to these post-translational modifications.

The monoclonal antibody of the invention may be prepared by conventional methods, such as the production of hybridomas as described by Koehler and Milstein (1975), immortalization of human B lymphocytes by Epstein-Barr's virus (EBV), or more recent ones, such as phage display technology, use of a combinatorial library of human or transgenic animal antibodies, notably from the mouse, and of course monoclonal antibodies may also be prepared by molecular engineering.

In a particular embodiment, the monoclonal antibody of the invention is prepared in genetically modified cells by introducing at least one vector allowing antibodies to be expressed, these cells being eukaryotic or prokaryotic cells, notably cells from non-human transgenic mammals, insects, plants, bacteria or yeasts. Advantageously, these cells are selected from rat myeloma cell lines, notably YB2/0 and IR983F, human myeloma lines such as Namalwa or any other cell of human origin such as PERC6, CHO lines, notably CHO-K, CHO-Lec10, CHO-Lec1, CHO Pro-5, CHO dhfr-, CHO Lec13, or other lines selected from Wil-2, Jurkat, Vero, Molt-4, COS-7, 293-HEK, BHK, K6H6, NSO, SP2/0-Ag 14 and P3X63Ag8. 653.

Preferably, the monoclonal antibody of the invention is monospecific, e.g. its Fab are directed to the same antigen.

The monoclonal antibody of the present invention may be a recombinant, chimeric, humanized or human antibody.

The term « chimeric antibody » as used herein is intended to include all immunoglobulin molecules comprising a human and non-human portion. More specifically, the antigen combining region (or variable region) of a chimeric antibody is derived from a non-human source (e.g., murine) and the constant region of the chimeric antibody (which confers biological effector function to the immunoglobulin) is derived from a human source. The chimeric antibody should have the antigen binding specificity of the non-human antibody molecule and the effector function conferred by the human antibody molecule. The variable region may be murine, from rats, rabbits, non-human primate, goat, and the like, without limitation.

In a preferred embodiment of the invention, the monoclonal antibody comprises a murine variable region, and a human constant region.

The term "humanized antibody" refers to an antibody that includes at least one humanized antibody chain (i.e., at least one humanized light or heavy chain). The term "humanized antibody chain" refers to an antibody chain (i.e., a light or heavy chain, respectively) having a variable region that includes a variable framework region substantially from an human antibody, and complementarity determining regions (CDRs) (e.g., at least one CDR, preferably two CDRs, more preferably three CDRs) substantially a non-human antibody, and further includes constant regions.

The term "human antibody" is intended to include an antibody which, when aligned to a human antibody amino acid sequence for comparison purposes, the region shares at least 80-90%, preferably 90-95%, more preferably 95-99% identity (i.e., local sequence identity) with the human framework or constant region sequence, allowing, for example, for conservative substitutions, consensus sequence substitutions, germline substitutions, backmutations, and the like. The introduction of conservative substitutions, consensus sequence substitutions, germline substitutions, backmutations, and the like, is often referred to as "optimization" of a humanized antibody or chain.

The term "human antibody" is also intended to include a human antibody which naturally exists in the human body, and antibodies obtained from a human antibody phage library and from a human antibody-producing transgenic animal or a human antibody-producing transgenic plant, which are prepared based on recent advances in genetic engineering, cell engineering and developmental engineering techniques.

In a preferred embodiment, chimeric, humanized and human antibodies are produced by recombinant expression. Nucleic acids encoding chimeric, humanized or human light and heavy chain variable regions, optionally linked to constant regions, are inserted into expression vectors. The light and heavy chains can be cloned in the same or different expression vectors. When heavy and light chains are cloned on separate expression vectors, the vectors are cotransfected to obtain expression and assembly of intact immunoglobulins. Once expressed, the whole antibodies, their dimers, individual light and heavy chains, or other immunoglobulin forms of the present invention can be purified according to standard procedures of the art, including ammonium sulfate precipitation, affinity columns, column chromatography, HPLC purification, gel electrophoresis and the like (see generally Scopes, Protein Purification (Springer-Verlag, N.Y., (1982)). Substantially pure immunoglobulins of at least about 90 to 95% homogeneity are preferred, and 98 to 99% or more homogeneity most preferred, for pharmaceutical uses.

The DNA segments encoding immunoglobulin chains are operably linked to control sequences in the expression vector(s) that ensure the expression of immunoglobulin polypeptides. Expression control sequences include, but are not limited to, promoters (e.g., naturally-associated or heterologous promoters), signal sequences, enhancer elements, and transcription termination sequences. Preferably, the expression control sequences are eukaryotic promoter systems in vectors capable of transforming or transfecting eukaryotic host cells. Once the vector has been incorporated into the appropriate host, the host is maintained under conditions suitable for high level expression of the nucleotide sequences, and the collection and purification of the crossreacting antibodies.

These expression vectors are typically replicable in the host organisms either as episomes or as an integral part of the host chromosomal DNA. Commonly, expression vectors contain selection markers (e.g., ampicillin-resistance, hygromycin-resistance, tetracycline resistance or neomycin resistance) to permit detection of those cells transformed with the desired DNA sequences (see, e.g., Itakura et al., U.S. Pat. No. 4,704,362).

Prokaryotic hosts particularly useful for cloning the polynucleotides (e.g., DNA sequences) of the present invention may be *E. coli,* bacilli, such as *Bacillus subtilus,* and other enterobacteriaceae, such as *Salmonella, Serratia,* and various *Pseudomonas* species. In these prokaryotic hosts, one can also MAK®e expression vectors, which will typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters will typically control expression, optionally with an operator sequence, and have ribosome binding site sequences and the like, for initiating and completing transcription and translation.

Other microbes, such as yeast, are also useful for expression. *Saccharomyces* is a preferred yeast host, with suitable vectors having expression control sequences (e.g., promoters), an origin of replication, termination sequences and the like as desired. Typical promoters include 3-phosphoglycerate kinase and other glycolytic enzymes. Inducible yeast promoters include, among others, promoters from alcohol dehydrogenase, isocytochrome C, and enzymes responsible for maltose and galactose utilization.

In addition to microorganisms, mammalian tissue cell culture may also be used to express and produce the polypeptides of the present invention (e.g., polynucleotides encoding immunoglobulins or fragments thereof). See Winnacker, From Genes to Clones, VCH Publishers, N.Y., N.Y. (1987). Eukaryotic cells are actually preferred, because a number of suitable host cell lines capable of secreting heterologous proteins (e.g., intact immunoglobulins) have been developed in the art, and include CHO cell lines, YB2/0 rat cell lines, various Cos cell lines, HeLa cells, preferably, myeloma cell lines, or transformed B-cells or hybridomas. Preferably, the cells are nonhuman. Expression vectors for these cells can include expression control sequences, such as an origin of replication, a promoter, and an enhancer (Queen et al., Immunol. Rev. 89:49 (1986)), and necessary processing information sites, such as ribosome binding sites, RNA splice sites, polyadenylation sites, and transcriptional terminator sequences. Preferred expression control sequences are promoters derived from immunoglobulin genes, SV40, adenovirus, bovine papilloma virus, cytomegalovirus and the like. See Co et al., J. Immunol. 148:1149 (1992).

Alternatively, antibody-coding sequences can be incorporated in transgenes for introduction into the genome of a transgenic animal and subsequent expression in the milk of the transgenic animal (see, e.g., Deboer et al., U.S. Pat. No. 5,741,957, Rosen, U.S. Pat. No. 5,304,489, and Meade et al., U.S. Pat. No. 5,849,992). Suitable transgenes include coding sequences for light and/or heavy chains in operable linkage with a promoter and enhancer from a mammary gland specific gene, such as casein or beta lactoglobulin.

The vectors containing the polynucleotide sequences of interest (e.g., the heavy and light chain encoding sequences and expression control sequences) can be transferred into the host cell by well-known methods, which vary depending on the type of cellular host. For example, calcium chloride transfection is commonly utilized for prokaryotic cells, whereas calcium phosphate treatment, electroporation, lipofection, biolistics or viral-based transfection may be used for other cellular hosts. (See generally Sambrook et al., Molecular Cloning: A Laboratory Manual (Cold Spring Harbor Press, 2nd ed., 1989)). Other methods used to transform mammalian cells include the use of polybrene, protoplast fusion, liposomes, electroporation, and microinjection (see generally, Sambrook et al., supra). For production of transgenic animals, transgenes can be microinjected into fertilized oocytes, or can be incorporated into the genome of embryonic stem cells, and the nuclei of such cells transferred into enucleated oocytes.

The monoclonal antibodies according to the invention have reduced immunogenicity, and long serum half-life.

Fcgamma receptors

Activated macrophages of the invention express on their cell membrane three types of Fcgamma receptors: FcgammaRI (CD64), FcgammaRII (CD32) and FcgammaRIII (CD16).

CD16 is also called "low affinity receptor", and is constitutively expressed on polymorphonuclear neutrophils (PMNs), monocytes and NK-cells. CD16 participates in various effector functions, notably phagocytosis of opsonized particles or of immune complexes, and in antibody-dependent cellular cytotoxicity (ADCC).

The monoclonal antibody of the kit of parts of the invention has a high affinity of its Fc region toward Fcgamma receptors present on activated macrophages, especially on CD16. More particularly, the invention describes the synergic interaction of particular monoclonal antibodies and activated macrophages. Contrary to the association of the state of the art of a conventional monoclonal antibody and an activated macrophage, in which the addition of blood serum in the medium inhibit the ADCC activity of the association, the blood serum has no influence on the ADCC of the association, i.e. of the kit of parts of the invention. This is due to the fact that the affinity of the Fc region of said monoclonal antibody to CD16 is higher than the affinity of the Fc region of the IgG present in the blood serum to the CD16. As a consequence, the ADCC activity in vivo of the kit of parts of the invention is almost the same as the ADCC activity measured in vitro in absence of blood serum.

Blood serum contains high concentrations of polyclonal IgG (also called "polyvalent IgG"). In vivo, ADCC activity is mediated via CD16 and CD64. It is known from the state of the art that polyvalent IgG can inhibit the mechanism of lysis of the effector cells via CD64, the CD64 being saturated by the addition of polyvalent IgG (see patent application WO 01/77181).

The applicant has shown that, surprisingly, the association in a kit of parts of monoclonal antibody whose affinity of the Fc region of said monoclonal antibody to CD16 is higher than the affinity of IgG present in the blood serum to the CD16 induces an ADCC activity that is not inhibited by the addition of blood serum in vitro.

Advantageously, the monoclonal antibody is therefore not displaced by IgG polyclonal antibodies in case of presence or addition of blood serum to the medium.

Due to the high affinity of the Fc region of said monoclonal antibody to CD16, the Fc region of the monoclonal antibody binds the CD16 of the activated macrophages, and this binding is not displaced by the polyclonal IgG, even present at high concentrations in the blood serum.

As a consequence, the kit of parts of the invention achieves optimal killing of the target cells even at low concentration of monoclonal antibody. Advantageously, the concentration of monoclonal antiboby of the kit of parts is inferior to the concentration of a conventional antibody of the same specificity, traditionally used alone to treat an autoimmune or a blood cancer.

Due to the high affinity of the Fc region of said monoclonal antibody to CD16, the kit of parts achieves target cell death even if the E:T ratio (effector cells to target cells ratio) is not necessarily high, which can be for example as low as 1:1. In an advantageous embodiment of the invention, the E:T ratio is comprised between 1:1 and 50:1.

In an advantageous embodiment of the invention, the Fc region of the monoclonal antibody of the invention has an association constant of at least $1.10^7$ $M^{-1}$ preferentially $1.10^8 M^{-1}$ or even $1.10^9 M^{-1}$ for CD16.

In a preferred embodiment of the invention, the concentration of monoclonal antibody in the kit of parts is inferior to 50 mg/billion MAK, preferably, 2.5 mg/ 100 million MAK.

In a particular embodiment of the invention, the monoclonal antibody binds to CD16 of said activated macrophages with an affinity of at least $1.10^7$ $M^{-1}$ preferably of $1.10^8 M^{-1}$ or even of $1.10^9$ $M^{-1}$ measured by Scatchard analysis or BIAcore technology.

For example, the monoclonal antibody of the invention can be prepared by means of the process described in patent application WO 01/77181. This process of preparing a monoclonal antibody capable of activating effector cells expressing CD16, and preferentially activated macrophages, comprises the following steps:

a) purifying several different monoclonal antibodies obtained from various cell lines or clones originating from cell lines selected from hybridomas, in particular heterohybridomas, and animal or human cell lines transfected with a vector comprising the gene encoding the antibody,
b) adding each monoclonal antibody obtained in step a) to a reaction mixture comprising:

i. the target cells of the monoclonal antibody,
ii. effector cells expressing CD16, preferably activated macrophages,
iii. polyvalent IgGs,

c) determining the percentage of lysis of the target cells and selecting the monoclonal antibodies which activate the effector cells causing significant lysis of effector cells (CD16-type ADCC activity).

The monoclonal antibody of the invention is in fact a composition containing antibodies, which are qualified as "monoclonal" because they originate from the same cell clone. All the antibodies of a composition of monoclonal antibody have the same amino acid sequence, and the same specificity. However, because of the post-translational modifications of eukaryote cells, all of the antibodies of one composition of monoclonal antibodies may not have the same glycan structure.

The antibodies of all human and animal subclasses have a N-oligosaccharide attached to the $CH_2$ domain of each heavy chain, at residue asparagine 297 (EU numbering) for human IgG. This asparagine (Asn) residue is also called "Fcγ glycosylation site", or "N-glycoside-linked sugar chain biding site", and the N-oligosaccharide is also called "N-glycoside-linked sugar chain".

The sugar chain terminus which binds to Asn297 is called a reducing end, and the opposite side is called a non-reducing end.

In the Fc region of an antibody of IgG type, two N-glycoside-linked sugar chain biding sites are present. So, two sugar chains are bound per one antibody molecule.

In a composition of monoclonal antibody, the N-glycoside-linked sugar chains have various structures, depending on the glycosylation specifics of the producing cell line, but it is known that they have a basic common core structure shown by the following structural formula :

← binding to the Asn297

■ : GlcNAc ● :Mannose

This core structure, common to all the antibodies of a same composition of monoclonal antibody, may comprise the following additional sugars: N-acetylglucosamine (GlcNAc), fucose (fuc), galactose (gal). The principal glycosylated forms of the N-oligosaccharide are shown below:

G0 G0F G1 G1F

Since the N-glycoside-linked sugar chain which binds to an antibody includes any sugar chain possibly containing one or several of these additional sugars, there are a number of combinations of sugar chains for the two N-glycoside sugar chains which bind to the antibody.

It has been shown surprisingly by the Applicant that the compositions of monoclonal antibody in which fucose content is less than 65%, or advantagesouly comprised between 15% and 45%, and preferably between 20% and 40%, exhibit a strong affinity of the Fc region for CD16. More particularly, this kind of composition of monoclonal antibody has an affinity of the Fc region of said antibody to CD16 higher than the affinity of IgG polyclonal antibodies to CD16. Moreover, the antibodies of such monoclonal antibody composition are not easily displaced by IgG present in blood serum.

A method of preparation of such a composition of monoclonal antibodies is given for example in the patent application WO 01/77181. In one embodiment of the invention, the composition of monoclonal antibody is produced in a cell which has a low fucose-adding enzyme activity to N-acetylglucosamine of the reducing terminal, wherein such enzyme may be fucosyltransferase.

In order to obtain a low fucose content glycosylated antibody, the skilled person may also use suitable enzymes such as endoglycosidases, including fucosidases.

In a preferred embodiment, the composition of monoclonal antibody of the invention is produced in YB2/0 (ATCC CRL-1662).

Advantageously, the monoclonal antibody of the kit of parts of the invention is directed against an antigen expressed by tumor cells or cells involved in auto-immune diseases

The term « antigen » as used herein designates an entity (e.g., a proteinaceous entity or peptide) to which an antibody specifically binds.

"Specific binding" of an antibody means that the variable region of the antibody exhibits appreciable affinity for antigen or a preferred epitope and, preferably, does not exhibit significant crossreactivity. "Appreciable" or preferred binding include binding with an affinity of at least $10^6$, $10^7$, $10^8$, $10^9$, or $10^{10}$ M$^{-1}$. Affinities greater $10^7$ M$^{-1}$, preferably greater than $10^8$ M$^{-1}$ are more preferred. An antibody that "does not exhibit significant crossreactivity" is one that will not appreciably bind to an undesirable entity (e.g., an undesirable proteinaceous entity). Preferably, specific binding is determined according to Scatchard analysis and/or competitive binding assays.

Preferably, the monoclonal antibody of the invention has specificity for the CD20 antigen, or in specific therefor.

CD20 is a cell surface antigen expressed on more than 90% of B-cell lymphomas, which does not shed or modulate in neoplastic cells (McLaughlin et al., J. Clin. Oncol. 16: 2825-2833 (1998b)). The CD20 antigen is a non-glycosylated, 35 kDa B-cell membrane protein involved in intracellular signaling, B-cell differentiation and calcium channel mobilization (Clark et al., Adv. Cancer Res. 52: 81-149 (1989); Tedder et al., Immunology Today 15: 450-454 (1994)).

The antigen appears as an early marker of the human B-cell lineage, and is ubiquitously expressed at various antigen densities on both normal and malignant B-cell populations. However, the antigen is absent on fully, mature B-cells (e.g., plasma cells), early B-cell populations and stem cells, making it a suitable target for antibody mediated therapy.

Such an anti-CD20 antibody, and its process of production, is described in the patent application WO2006/064121, whose content is incorporated herein by reference.

Advantageously, the heavy chain amino acid sequence of this monoclonal antibody is the sequence SEQ ID NO: 1.

Advantageously, the light chain amino acid sequence of this monoclonal antibody is the sequence SEQ ID NO: 2 or the sequence SEQ ID NO: 3.

Briefly, this antibody may be obtained, as described in the patent application, WO2006/064121 which is incorporated herein by reference, by means of the transfection of an appropriate cell, preferably YB2/0, by vectors permitting the expression of the heavy and the light chains described above.

In a preferred embodiment, the composition of monoclonal anti-CD20 has a fucose content of less than 65%, and preferentially comprised between 20 and 40%.

In a preferred embodiment of the invention, the monoclonal antibody of the kit of parts of the invention is produced by the clone R509 which is deposited at the CNCM under the accession number CNCM I-3314 (Collection Nationale de Cultures de Microorganismes, Institut Pasteur, 25 rue du Docteur Roux, 75724 Paris Cedex 15 - France).

In another preferred embodiment, the monoclonal antibody of the kit of parts of the invention is produced by the clone R603 which is deposited at the CNCM under the accession number CNCM I-3529.

In a preferred embodiment of the invention, the kit of parts of the invention contains the monoclonal antibody produced by the clone R603 or by the clone R509, and the activated macrophages MAK® (IDM, Paris, France).

The Applicant has shown that the kit of parts of the invention efficiently killed ex vivo Chronic Lymphoid Leukaemia cells isolated from cancer patients, even at 1 to 1 effector to tumor ratio, at low monoclonal antibody concentration (0.025 mg/million MAK), and in the presence of 50 % blood serum.

The kit of parts of the invention so achieves an optimal destruction of the target cells recognized by the variable region of the antibodies, due to the physical interaction between MAK® and anti-CD20 antibody. In contrast to what is seen with other anti-CD20 antibodies, the potent antitumoral activity is not inhibited by polyclonal IgG. This synergistic activity of the binary complex of macrophages and selected antibodies allows for greater potency in antitumoral activity in vivo, where normally most of the activity is inhibited by the IgG present in blood serum. In the case of chronic lymphocytic leukaemia (CLL), the injection of MAK and the selected anti CD-20 results in the destruction of residual leukaemic cells which could not be achieved by MAK coupled to other anti-CD20 antibodies such as Rituximab, or by the antibodies alone, even at very high doses, which compete with serum IgG for the Fc receptors. It was suggested that cross-linking the tumor CD-20 units via the Fc receptors of the macrophage membrane induced apoptosis of the tumor cells (Shan, Ledbetter and Press, 1998, Apoptosis of malignant human B cells by ligation of CD-20 with antibodies, Blood, 91, 1644).

Advantageously, the concentration of the monoclonal antibody in the kit of parts of the invention for the treatment of B-CLL can be less than 375 mg/m$^2$, and advantageously less than 20 mg/m$^2$.

Regarding its advantages in term of cytotoxicity, specificity and doses of the administered active substances, the kit of parts of the invention may be used for the treatment of any of the following diseases or disorders, where the disease or disorder is selected from the group consisting of an immune dysregulation disease, organ graft rejection and graft versus host disease. The malignant disease or disorder is selected from the group consisting of a hematopoietic tumor (lymphoma, leukemia, myeloma and the like). The B-cell malignancy is selected from the group consisting of indolent forms of B-cell lymphomas, aggressive forms of B-cell lymphomas, chronic lymphatic leukemias, acute lymphatic leukemias, Waldenstrom's macroglobulinemia, and multiple myeloma.

Regarding its advantages in term of cytotoxicity, specificity and doses of the administered active substances, the kit of parts of the invention may be used for the treatment of non-malignant B-cell disorders and related diseases, such as many autoimmune and immune dysregulatory diseases, including septicemia and septic shock among immune dysregulatory diseases. Auto-immune and/or inflammatory diseases can be systemic or organ specific and associated or not with pathogenic auto-antibodies. Examples of autoimmune and/or inflammatory disorders include, but are not limited to, Hashimoto's thyroiditis, pernicious anemia, sarcoidosis, primary biliary cirrhosis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, rapidly progressive glomerulonephritis, psoriasis, fibrosing alveolitis, ankylosing spondylitis, Goodpasture's syndrome, thromboangitis obliterans, ulcerative colitis, erythema multiforme, IgA nephropathy, polyarteritis nodosa, Henoch-Schonlein purpura, post-streptococcal nephritis, erythema nodosum, Takayasu's arteritis, Addison's disease, diabetes mellitus, rheumatoid arthritis, systemic lupus erythematosus, Sjogren's syndrome, Sydenham's chorea, lupus nephritis, rheumatic fever, polyglandular syndromes, bullous pemphigoid, multiple sclerosis, autoimmune inner ear disease, myasthenia gravis, Reiter's syndrome, Graves disease, auto-immune cytopenia and other adult and children haematological complications such as auto-immune anemia, cold agglutinin disease, acute and chronic idiopathic thrombopenic purpura, acquired auto-immune hemophilia, children systemic auto-immune diseases including the antiphospholipide syndrome, chronic juvenile arthritis or inflammatory myositis.

Preferably, the diseases that can be treated with the kit of parts of the invention are those where the level of effector cells is diminished or where the functionality of effector cells is diminished.

Preferably, the kit of parts of the invention is used as an autologous therapy. In one embodiment of the invention, the present treatment consists in the local or systemic injection of autologous activated macrophages (preferentially MAK® killer cells) which have access to injured areas, and in particular to hypoxic areas, where they tend to concentrate and of monoclonal antibody according to the invention, linked to the MAK before the injection.

According to an advantageous embodiment of the invention, in the combined preparation of the invention (i.e. the kit of parts), the activated macrophages and the monoclonal antibody are in the form of injectable solutions.

In another advantageous embodiment of the invention, in the combined preparation, the injectable solutions are in the form of locally injectable solutions.

In another advantageous embodiment in the combined preparation of the invention, the injectable solutions are in the form of systemically injectable solutions.

Conveniently, the kit of parts formed of antibodies associated to MAK cells are kept frozen in separate aliquoted vials in the presence of 4% human serum albumin and of 10% DMSO, at -80°C until use.

Advantageously, 6 administrations are carried out on the patient (3 to 10 administrations over a two year period).

In another embodiment of the invention, the activated macrophages are to be administered at a dose of about $10^8$ activated macrophages per injection.

In another embodiment of the invention, the monoclonal antibody is to be administered at a dose of about 2.5 mg antibody per injection if linked to the MAK® prior to injection.

In another advantageous embodiment of the invention, the activated macrophages are to be administered in a repeated way up to ten times, the interval between each administration being between three days to three months.

In another advantageous combined preparation of the invention, the monoclonal antibody and the activated macrophages are to be injected simultaneously. For example, they may be administered in the same solution, or in a different one, but at the same time. For example, the activated macrophages may be administered in a right arm, whilst at the same time the monoclonal antibody may be administered in the left arm.

In another advantageous combined preparation of the invention, the monoclonal antibody and the activated macrophages are to be administered in a sequential manner, the monoclonal antibody being administered before the activated macrophages. For example, the monoclonal antibody is administered in one arm, for example the left one, before the macrophage, which is administered in the other arm.

In another advantageous combined preparation of the invention, the activated macrophages and the monoclonal antibody are to be administered sequentially, the activated macrophages being administered before the monoclonal antibody. For example, monoclonal antibody is administered in one arm, for example the left one, before the macrophage, which is administered in the other arm.

In another advantageous combined preparation of the invention, the time interval between the administration of activated macrophages and administration of monoclonal antibody is of one day to two weeks.

More preferably, activated macrophages and antibodies are associated in vitro at room temperature and then kept frozen at - 80°C until injection.

In another embodiment, the kit of parts of the invention can be administered after the treatment of the patient by a conventional therapy, e.g. by chemotherapeutic agents.

This treatment can be conducted after first failure and relapse following conventional chemotherapies, or before chemotherapy, to prevent chemoresistance. Local treatment with chemotherapy drugs causes cell necrosis and release of chemokines which call and actively recrute macrophages and monocyte derived cells. Therefore, combining conventional chemotherapy with the immunotherapy based on the kit of parts of the invention can synergistically increase cytotoxicity and increase immune response at the same time as preventing the establishment of resistance. Additionally to a first treatment combining conventional approach with kit of parts immunotherapy, kit of parts adoptive therapy can be proposed after failure and relapse.

The active ingredients which are administered either at the same time, or separately, or sequentially, according to the invention, do not represent a mere aggregate of known agents, but a new combination with the surprising valuable property described above.

It is to be stressed that the combined preparation also designated by a kit-of-parts means that the components of the combined preparation are not necessarily present as a union e.g. in composition, in order to be available for separate or sequential application.

A particularly effective way to use the kit of parts is to combine the parts *in vitro,* to freeze the resulting cell drug until use, allowing all quality controls before batch release.

Another object of the invention is a pharmaceutical composition comprising the kit of parts of the invention.

Another object of the invention is the use of a kit of parts of the invention for the preparation of a medicament for the treatment of a hematopoietic tumor or an autoimmune disease.

The medicament may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, etc. The compositions may be sterilised by conventional, well-known sterilisation techniques.

Preferably, the formulation is in the form of 3 to 15 flasks of 10 ml each, containing 200 million MAK and 2.5 mg of antibodies, resuspended in 4 % of HSA (human serum albumin) and 10% of DMSO (dimethylsulfoxide) and frozen at - 80°C. Following safety controls, the product is released, and each dose can be thawed and diluted in HSA 4% prior to injection.

DESCRIPTION OF THE FIGURES

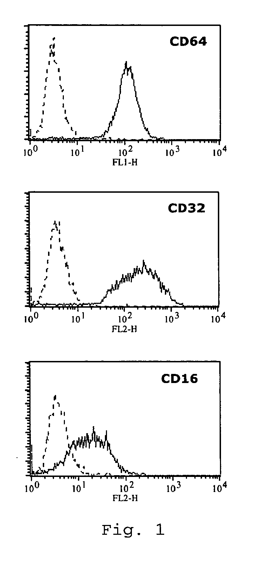

Figure 1: Fc expression by MAK® cells from HD497 (one representative healthy donor). Isotype controls are shown as dotted lines and specific labelling as solid lines.

## **EXAMPLES**

### **Example 1 : preparation of MAK**

Peripheral blood mononuclear cells (PBMC) were isolated using a COBE Spectra cytapheresis system to collect $10^{10}$ mononuclear cells in approximately three hours, then were transferred to the IDM cell-processing center. Macrophages were obtained from peripheral monocytes using the MAK-cell technology ((Immunobiology 210 (2005) 267-277: Anti-tumor properties of human-activated macrophages produced in large scale for clinical application ; Veronique Baron-Bodo, Paula Doceur, Marie-Laure Lefebvre, Karine Labroquere, Catherine Defaye, Christophe Cambouris, Didier Prigent, Margarita Salcedo,Aure'lie Boyer, Alessandra Nardin) IDM, Paris, France), and also Thiounn N, Pages F, Mejean A, et al: Adoptive immunotherapy for superficial bladder cancer with autologous macrophage activated killer cells. J. Urol. 168:2373-2376, 2002 ; De Gramont A, Gangji D, Louvet C, et al: Adoptive immunotherapy of ovarian carcinoma. Gynecol. Oncol. 86:102-103, 2002). The MAK-cells are prepared according to GMP procedures by culture at a concentration of approximately 5 x $10^6$/mL in Iscove modified Dulbecco medium devoid of any animal or human proteins, and supplemented with granulocyte-macrophage colony stimulating factor (GM-CSF) (500 IU/mL, Novartis, France) and 2% of autologous serum. Cell culture was carried out at 37°C for six days in a humidified incubator under 5% of $CO_2$, in hydrophobic EVA bags. After six days of culture, IFN-$\gamma$ (Boehringer Ingelheim, France) was added to the culture containers at the dose of 250 U/mL. On the seventh day of culture, macrophages were purified by an elutriation in physiological solution. The number of macrophages collected was at least $10^9$ per culture. They were eventually combined with monoclonal antibodies by 1 h contact at room temperature and aliquoted at 200 million cells per vial and frozen at -80°C in the presence of 4% Human Serum Albumin and 10% DMSO.

They were characterized microscopically and by assessment of surface antigens, in particular, CD14, CD64 and HLA-DR, and for their anti-tumoral properties on tumor cell targets. Sterility was controlled before batch release.

Fc expression by MAK cells from HD497

To evaluate cell surface marker expression MAK cell preparations were incubated in presence of various monoclonal antibodies and resuspended in TO-PRO-3 (Molecular Probes) solution. Stained cells were further acquired using a FACSCalibur flow cytometer. TO-PRO-3 dye allows exclusion of dead cells during acquisition. Samples were analyzed by flow cytometry by gating on the viable (TO-PRO-3) cell population of interest (FSC/SSC).

Table 1: Monoclonal Ab used in the staining experiment

| Ab | Dye | Clone | Isotype | Supplier |
|---|---|---|---|---|
| IgG1 | FITC | 679.1Mc7 | NA | Immunotech |
| CD64 | FITC | 22 | IgG1 | Immunotech |
| IgG2a | FITC | 7T4-1F5 | NA | Immunotech |
| CD32 | PE | 2E1 | IgG2a | Immunotech |
| IgG1 | PE | 679.1Mc7 | NA | Immunotech |
| CD16 | PE | 3G8 | IgG1 | Immunotech |

Figure 1 shows the molecule cell surface expression by MAK. Day 7 activated and elutriated macrophages from healthy donors were stained with antibodies specific for CD64, CD32 and CD16. Cells were analyzed by flow cytometry. Isotype controls are shown as dotted lines and specific labelling as solid lines. One representative donor HD497 is presented here.

**Example 2: Manufacture of anti-CD20 antibody for inclusion in the kit of parts of the invention.**

The monoclonal anti-CD20 antibodies with high affinity for Fc gamma receptors EMAB6 (produced by the cell line R509, deposited at the CNCM under the accession number I-3314) and EMAB603 (produced by the cell line R603, deposited at the CNCM under the accession number 1-3529) were prepared as described in patent WO 2006/064121, whose content is incorporated herein by reference, in particular pages 26-33. EMAB6 and EMAB603 are also named "LFB-antibodies" or "EMABling antibodies".

Briefly, the sequences of the variable regions of the murine antibody CAT-13.6E12 (desposited at the Deutsche Sammlung von Mikroorganismen und zellkulturen GmbH (DSMZ) under the accession number ACC 474) were determined.

Then, the expression vectors of the heavy and of the light chain of the monoclonal antibodies EMAB6 and EMAB603 were constructed. The light chain variable sequence was chimerized with a constant light chain sequence and cloned in an expression vector. The same protocol was applied for the chimerization of the heavy chain.

Then, cell line YB2/0 is transfected with the two vectors expressing the heavy and the light chains of the antibody EMAB6 and EMAB603.

The structure of the N-glycan of the heavy chain of EMAB6 and EMAB603 antibodies was analysed by HPCE-LIF (high performance capillary electrophoresis with detection of laser induced fluorescence).

The anti-CD20 monoclonal antibody was desalted on a Sephadex G-25 column (HiTrap Desalting, Amersham Biosciences), dried by evaporation and put in suspension in a hydrolysis buffer of PNGase F (Glyko) in presence of 50 mM de β-mercaptoéthanol. After 16 h of incubation at 37°C, the protein fraction was precipitated by addition of absolute ethanol and the supernatant, which contain the N-glycans, was dried by evaporation. The oligosaccharides thus obtained were labelled directly by a fluorochrome : the APTS (1-amino-pyrène-3,6,8-trisulfonate), or submitted to the action of specific exoglycosidases before being labelled by the APTS. The oligosaccharides thus labelled were injected on a cappilary N-CHO, separated and quantified by HPCE-LIF.

The evaluation of fucose content was carried out either by addition of isolated fucosylated forms, or more specifically after simultaneous action of neuraminidase, β-galactosidase and N-acetylhexosaminidase, leading to 2 peaks on the electrophoresis which correspond to the fucosylated or unfucosylated pentasaccharide [GlcNac2-Man3].

Table 1 : analysis of N-glycans of the antibody

| Anti-CD20 | % Fucose | % Galactose | Fuc/Gal |
|---|---|---|---|
| EMAB603 | 15 | 37 | 0,4 |

The content of fucose, expressed in percentage, was calculated using the following formula :

$$\text{Content of fucose} = \frac{[\text{GlcNac2-Man3] fucosylated} \times 100}{[\text{GlcNac2-Man3} + \text{GlcNac2-Man3 fucosylated}]}$$

The content of galactose, expressed in percentages, was calculated by adding the percentages of the oligosaccharide forms containing galactose, obtained after the action of neuraminidase and fucosidase. The formula used was the following : Content of galactose = (G1+G1B) + 2x(G2+ G2B)

The ratio content of fucose/content of galactose was obtained by dividing the content of fucose by the content of galactose.

**Example 3: Antitumoral efficacy of the kit of parts: in vitro experiments**

MAK cells are armed with increasing doses of rituximab or with the anti-CD20 antibody according to the invention and cultured for up to 20h with CLL tumor cells or Raji Burkitt Lymphoma cell line (ATCC-CLL-86) in absence or in presence of up to 50% blood serum. Following incubation, a flow cytometry assay allows quantification of the remaining alive tumor cells. Briefly, macrophages and tumor cells are stained with specific antibodies and acquired on the flow cytometer in presence of a dead cell exclusion dye together with an internal beads control allowing cell quantification. Cells are analyzed on the flow cytometer and the percentage of cell kill was calculated.

% cell kill = [(number of alive CLL in the absence of MAK + Ab)-(number of alive CLL in the presence of MAK + Ab)/ (number of alive CLL in the absence of MAK + Ab)] x 100

At a macrophage/tumor ratio of 1/1 and with 0.001 mg antibody added to the macrophages, a 70% kill can be achieved with rituximab combined to MAK.

When the same experiment is conducted in the presence of 50% blood serum, the combined MAK-rituximab effector gives only less than 10% of tumor kill.

In contrast, when the combined MAK-antiCD20 Ab EMAB6 according to the invention is used, the effective kill is not markedly reduced by the addition of 50% blood serum.

Conclusion from examples 2 and 3: *In vitro* antitumoral efficacy of MAK cells combined to anti-CD20 antibody with blood (rituximab vs according to the invention).

Compared to MAK-rituximab, cytotoxicity is fully maintained in presence of the MAK- antiCD20 antibody effector. This result suggests that the new binary complex (MAK + anti-CD20 antibody according to the invention) can achieve new very potent antitumoral effects even in the presence of IgG polyclonal antibodies present in blood serum.

**Example 4 : Experimental murine model (SCID mice grafted with tumors expressing CD20) showing anti-tumor efficacy of the new binary complex MAK-Ab (according to the invention)**

SCID mice were administered cells from B-CLL patients labelled ex *vivo* with a fluorescent dye (CFSE). The mice then received the MAK cells previously combined to anti-CD20 antibody (rituximab or high CD16 affinity antibody = EMABling according to the invention) and the clearance of the tumor cells is followed in the blood of the mice as the decrease of the number of circulating fluorescent cells (using flow cytometry technology). The percent of tumor cells is monitored in different organs such as the spleen, the liver, the kidney. The absence of effect of injection of human serum on the anti-tumor efficacy is also shown. Different E/T ratios and administration route of the therapeutic product.

**Example 5: Infusion of MAK combined to anti-CD20 antibodies to patients with chronic lymphoid leukaemia.**

The survival expectancy of patients with advanced chronic lymphocytic leukemia (CLL) is usually short. It depends on the duration of response to chemotherapy, and to the level of persistent minimal residual disease (MRD). The aim of the study was to assess the efficacy on MRD and safety of autologous macrophage-activated killer cells (MAK®) associated to anti-CD20 antibody in MRD-positive, CLL patients (Chahoui, Maloum, Cazin, Azar, Chokri, Prigent, 2007 Molecular remission in CLL patients with minimal residual disease: phase 2 trial of autologous activated macrophages armed ex vivo with humanized anti-CD20 monoclonal antibodies).

The number of MAK/rituximab administered was based on previously published experience with MAK cells.

Six infusions of MAK/anti-CD-20Ab were organised. To prevent transfusional reactions, patients were premedicated with intravenous dexchlorpheniramine. Paracetamol could be administered over 24 hours after infusion if a fever occurred. Corticosteroids were prohibited.

*Patients and Methods:* Heavily pretreated MRD-positive CLL patients were enrolled in a phase II trial to receive infusions of MAK®/anti-CD20. Mononuclear cells were isolated from peripheral blood by cytapheresis. Activated macrophages were obtained after 7 days of differentiation and activation with gamma interferon before purification by apheresis (Immuno-Designed Molecules, Paris, France). MAKs were then incubated for 1 h at room temperature with rituximab or with the EMAB6 antib-CD20 antibody according to the invention. The preparation was then aliquoted in vials (containing 200 million MAK having fixed 2.5 mg anti-CD20), frozen in the presence of 4% human serum albumin and 10% DMSO, and kept at -80°C until its reinfusion intravenously. MRD assessments were performed using immunophenotyping, standard polymerase chain reaction (PCR), and clone-specific PCR.

*Results:* The six injections were completed in 80% of the patients enrolled with MAK-Rituximab without significant adverse events. Several patients achieved a durable immunophenotypic, and molecular remission. After a median-follow-up of 60 months, some patients were still in complete phenotypic plus molecular remission, and in complete clinical remission. The median progression-free survival was more significantly increased in patients receiving MAK plus EMEAB6 than in patients receiving MAK-Rituximab.

**Claims**

1. A kit of parts for the treatment of cancer or infectious diseases, comprising activated macrophages and a monoclonal antibody, wherein the affinity of the Fc region of said antibody to Fc gamma receptor III (CD16) is higher than the affinity of IgG polyclonal antibodies to said Fc gamma receptor III.

2. A kit of part according to claim 1, wherein as a result of their high affinity of the Fc region of said antibody to CD16, said monoclonal antibody is not displaced by IgG polyclonal antibodies, especially by IgG present in blood serum.

3. A kit of parts according to claim 1 or 2, wherein said monoclonal antibody binds to CD16 of said macrophage with an affinity of at least $2.10^6$ M$^{-1}$, more advantageously of at least $1.10^7$ M$^{-1}$, or preferably of $1.10^8$ M$^{-1}$ or even of $1.10^9$ M$^{-1}$, as determined by Scatchard analysis.

4. A kit of parts according to anyone of claims 1 to 3, wherein the monoclonal antibody is produced in the form of a composition of monoclonal antibodies, each of said monoclonal antibody having N-glycoside-linked sugar chains bound on the Fcγ glycosylation site (Asn 297, EU numbering), and wherein among said N-glycoside-linked sugar chains of all the antibodies of the composition, the fucose content is less than 65%.

5. A kit of parts according to anyone of the preceding claims, wherein said activated macrophage is a MAK®.

6. A kit of parts according anyone of the preceding claims, wherein said antibody has a variable region directed against an antigen expressed by tumors cells or involved in auto-immune diseases.

7. A kit of parts according to claim 6, wherein said antigen is CD20.

8. A kit of parts according to claim 7, wherein said monoclonal antibody is EMAB6, produced by the clone R509, deposited to the CNCM under the accession number I-3314.

9. A kit of parts according to claim 7, wherein said monoclonal antibody is EMAB603, produced by the clone R603, deposited to the CNCM under the accession number I-3529.

10. A kit of parts according to anyone of the preceding claims, wherein said cancer is chronic lymphoid leukaemia (CLL).

**11.** Use of a kit of parts as defined in anyone of the preceding claims, for the preparation of a medicament.

**12.** Use of a kit of parts as defined in anyone of the preceding claims, for the preparation of a medicament for the treatment of hematopoietic tumor or autoimmune disease.

**13.** Use according to claim 12, wherein said hematopoietic tumor is selected from leukemia, lymphoma and myeloma.

**14.** Use according to the claim 12, wherein said hematopoietic tumor is selected from the group consisting of indolent forms of B-cell lymphomas, aggressive forms of B-cell lymphomas, chronic lymphatic leukemias, acute lymphatic leukemias, Waldenstrom's macroglobulinemia and multiple myeloma.

**15.** Use according to claim 12, wherein said autoimmune disease is selected from immune dysregulation disease, inflammatory disorders, organ graft rejection or graft versus host disease, septicemia and septic shock, Hashimoto's thyroiditis, pernicious anemia, sarcoidosis, primary biliary cirrhosis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, rapidly progressive glomerulonephritis, psoriasis, fibrosing alveolitis, ankylosing spondylitis, Goodpasture's syndrome, thromboangitis obliterans, ulcerative colitis, erythema multiforme, IgA nephropathy, polyarteritis nodosa, Henoch-Schonlein purpura, post-streptococcal nephritis, erythema nodosum, Takayasu's arteritis, Addison's disease, diabetes mellitus, rheumatoid arthritis, systemic lupus erythematosus, Sjogren's syndrome, Sydenham's chorea, lupus nephritis, rheumatic fever, polyglandular syndromes, bullous pemphigoid, multiple sclerosis, autoimmune inner ear disease, myasthenia gravis, Reiter's syndrome, Graves disease, auto-immune cytopenia and other adult and children haematological complications such as auto-immune anemia, cold agglutinin disease, acute and chronic idiopathic thrombopenic purpura, acquired auto-immune hemophilia and children systemic auto-immune diseases including the antiphospholipide syndrome, chronic juvenile arthritis or inflammatory myositis.

**16.** Pharmaceutical preparation containing a kit of parts as defined in anyone of claims 1 to 10.

Fig. 1

**European Patent Office**

# EUROPEAN SEARCH REPORT

Application Number

EP 07 29 0522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | WO 2006/064121 A (FRACTIONNEMENT ET DES BIOTECHN [FR]; DE ROMEUF CHRISTOPHE [FR]; GAUCHE) 22 June 2006 (2006-06-22) <br> * the whole document * <br> ----- | 1-16 | INV. <br> C07K16/28 <br> A61K35/14 <br> A61K39/395 <br> A61P35/00 |
| Y | WO 2006/085967 A (XENCOR INC [US]; DAHIYAT BASSIL I [US]; LAZAR GREGORY ALAN [US]; VAFA) 17 August 2006 (2006-08-17) <br> * the whole document * <br> ----- | 1-16 | |
| Y | LAZAR G A ET AL: "Engineered antibody Fc variants with enhanced effector function" PROCEEDINGS OF THE NATIONAL ACADEMY OF SCIENCES OF USA, NATIONAL ACADEMY OF SCIENCE, WASHINGTON, DC, US, vol. 103, no. 11, March 2006 (2006-03), pages 4005-4010, XP002403708 ISSN: 0027-8424 * the whole document * <br> ----- | 1-16 | |
| Y | IIDA SHIGERU ET AL: "Nonfucosylated therapeutic IgG1 antibody can evade the inhibitory effect of serum immunoglobulin G on antibody-dependent cellular cytotoxicity through its high binding to FcgammaRIIIa" CLINICAL CANCER RESEARCH, THE AMERICAN ASSOCIATION FOR CANCER RESEARCH, US, vol. 12, no. 9, 1 May 2006 (2006-05-01), pages 2879-2887, XP002432763 ISSN: 1078-0432 * the whole document * <br> ----- <br> -/-- | 1-16 | TECHNICAL FIELDS SEARCHED (IPC) <br><br> A61K <br> A61P <br> C07K |

The present search report has been drawn up for all claims

| Place of search | Date of completion of the search | Examiner |
|---|---|---|
| The Hague | 21 September 2007 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 07 29 0522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SHINKAWA T ET AL: "The absence of fucose but not the presence of galactose or bisecting N-acetylglucosamine of human IgG1 complex-type oligosaccharides shows the critical role of enhancing antibody-dependent cellular cytotoxicity" JOURNAL OF BIOLOGICAL CHEMISTRY, AMERICAN SOCIETY OF BIOLOCHEMICAL BIOLOGISTS, BIRMINGHAM,, US, vol. 278, no. 5, 31 January 2003 (2003-01-31), pages 3466-3473, XP002355427 ISSN: 0021-9258 * the whole document * ----- | 1-16 | |
| Y | SUTTON LAURENT ET AL: "Intravenous infusion of activated autologous macrophages armed with anti-CD20 monoclonal antibodies can yield sustained molecular remission in CLL" BLOOD, vol. 98, no. 11 Part 1, 16 November 2001 (2001-11-16), page 808a, XP008083776 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971 * the whole document * ----- | 1-16 | TECHNICAL FIELDS SEARCHED (IPC) |

-/--

The present search report has been drawn up for all claims

| Place of search | Date of completion of the search | Examiner |
|---|---|---|
| The Hague | 21 September 2007 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**EUROPEAN SEARCH REPORT**

Application Number

EP 07 29 0522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHAPEROT L ET AL: "DIFFERENTIATION OF ANTIGEN-PRESENTING CELLS (DENDRITIC CELLS AND MACROPHAGES) FOR THERAPEUTIC APPLICATION IN PATIENTS WITH LYMPHOMA" LEUKEMIA, MACMILLAN PRESS LTD, US, vol. 14, no. 9, September 2000 (2000-09), pages 1667-1677, XP001037401 ISSN: 0887-6924 * the whole document * | 1-16 | |
| Y | UCHIDA J ET AL: "The innate mononuclear phagocyte network depletes B lymphocytes through Fc receptor-dependent mechanisms during anti-CD20 antibody immunotherapy" JOURNAL OF EXPERIMENTAL MEDICINE, TOKYO, JP, vol. 199, no. 12, 21 June 2004 (2004-06-21), pages 1659-1669, XP003002436 ISSN: 0022-1007 * the whole document * | 1-16 | |
| | | | **TECHNICAL FIELDS SEARCHED (IPC)** |
| A | MANCHES OLIVIER ET AL: "In vitro mechanisms of action of rituximab on primary non-Hodgkin lymphomas." BLOOD 1 FEB 2003, vol. 101, no. 3, 1 February 2003 (2003-02-01), pages 949-954, XP002451263 ISSN: 0006-4971 * the whole document * | 1-16 | |

-/--

The present search report has been drawn up for all claims

| Place of search | Date of completion of the search | Examiner |
|---|---|---|
| The Hague | 21 September 2007 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 07 29 0522

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PREITHNER ET AL: "High concentrations of therapeutic IgG1 antibodies are needed to compensate for inhibition of antibody-dependent cellular cytotoxicity by excess endogenous immunoglobulin G" MOLECULAR IMMUNOLOGY, ELMSFORD, NY, US, vol. 43, no. 8, March 2006 (2006-03), pages 1183-1193, XP005245960 ISSN: 0161-5890 * the whole document * | 1-16 | |
| D,A | LEFEBVRE MARIE-LAURE ET AL: "Ex vivo-activated human macrophages kill chronic lymphocytic leukemia cells in the presence of rituximab: mechanism of antibody-dependent cellular cytotoxicity and impact of human serum." JOURNAL OF IMMUNOTHERAPY (HAGERSTOWN, MD. : 1997) 2006 JUL-AUG, vol. 29, no. 4, July 2006 (2006-07), pages 388-397, XP008083749 ISSN: 1524-9557 * the whole document * | 1-16 | TECHNICAL FIELDS SEARCHED (IPC) |
| A | HERVÉ FRIDMAN, JEAN-FRANÇOIS PROST, PASCAL BRETON, SÉBASTIEN AMIGORENA: "Transversales Santé du 21 novembre 2006 au Centre des Cordeliers" 21 November 2006 (2006-11-21), XP002451264 Retrieved from the Internet: URL:http://www.parisdeveloppement.com/file admin/user_upload/animation/Transvesrales/ Syntheses/Synthese_Transversale_novembre_0 6.pdf> [retrieved on 2007-09-14] * the whole document * | 1-16 | |

The present search report has been drawn up for all claims

| Place of search | Date of completion of the search | Examiner |
|---|---|---|
| The Hague | 21 September 2007 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 985 633 A1**

## ANNEX TO THE EUROPEAN SEARCH REPORT
## ON EUROPEAN PATENT APPLICATION NO.

EP 07 29 0522

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2007

| Patent document cited in search report | | Publication date | Patent family member(s) | | Publication date |
|---|---|---|---|---|---|
| WO 2006064121 | A | 22-06-2006 | AU<br>CA<br>EP<br>FR | 2005315534 A1<br>2590303 A1<br>1824887 A2<br>2879204 A1 | 22-06-2006<br>22-06-2006<br>29-08-2007<br>16-06-2006 |
| WO 2006085967 | A | 17-08-2006 | NONE | | |

For more details about this annex : see Official Journal of the European Patent Office, No. 12/82

EPO FORM P0459

28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5843439 A
- US 5776456 A
- US 5736137 A
- WO 9426875 A
- EP 0806959 A
- WO 8702671 A, Liu
- US 4704362 A, Itakura
- US 5741957 A, Deboer
- US 5304489 A, Rosen
- US 5849992 A, Meade
- WO 0177181 A
- WO 2006064121 A

### Non-patent literature cited in the description

- **GERBER J. S. et al.** *Microbes and Infection,* 2001, vol. 3, 131-139
- **BILLADEAU D. D. et al.** *The Journal of Clinical Investigation,* 2002, vol. 2 (109), 161-1681
- **RAVETCH J. V. et al.** *Annu. Rev. Immunol.,* 2001, vol. 19, 275-90
- **ROBBINS ; ANGELL.** Basic Pathology. W.B. Saunders Co, 1976, 68-122
- Principles of Cancer Patient Management. **STOCKDALE.** Scientific American: Medicine. 1998, vol. 3
- **GREEN M. C. et al.** *Cancer Treat Rev.,* 2000, vol. 26, 269-286
- **WEINER L M.** *Semin Oncol.,* 1999, vol. 26 (14), 43-51
- **REFF et al.** *Blood,* 1994, vol. 83, 435-445
- **KENNEDY AD ; BEUM PV ; SOLGA MD et al.** Rituximab infusion promotes rapid complement depletion and acute CD20 loss in chronic lymphocytic leukaemia. *J. Immunol.,* 2004, vol. 172, 3280-3288
- **HILMEN P.** Advancing therapy for chronic lymphocytic leukaemia- the role of rituximab. *Semin Oncol.,* 2004, vol. 31, 22-26
- Activation of macrophages for tumor cell kill: effector mechanism and regulation. **ADAMS D. ; HAMILTON T.** Macrophages and cancer. CRC Press, 1988, 27
- **FIDLER I.** Macrophages and metastases. A biological approach to cancer therapy. *Cancer Res.,* 1985, vol. 45, 4714
- **LEFEBVRE ML ; STEFAN W. KRAUSE ; SALCEDO M ; NARDIN A.** Ex vivo activated human macrophages kill chronic lymphocytic leukemia cells in the presence of Rituximab: mechanism of antibody-dependent cellular cytotoxicity and impact of human serum. *J. Immunother,* 2006, vol. 29 (4), 388-397
- *Immunobiology,* 2005, vol. 210, 267-277
- **WINNACKER.** From Genes to Clones. VCH Publishers, 1987
- **QUEEN et al.** *Immunol. Rev.,* 1986, vol. 89, 49
- **CO et al.** *J. Immunol.,* 1992, vol. 148, 1149
- **SAMBROOK et al.** Molecular Cloning: A Laboratory Manual. Cold Spring Harbor Press, 1989
- **MCLAUGHLIN et al.** *J. Clin. Oncol.,* 1998, vol. 16, 2825-2833
- **CLARK et al.** *Adv. Cancer Res.,* 1989, vol. 52, 81-149
- **TEDDER et al.** *Immunology Today,* 1994, vol. 15, 450-454
- **THIOUNN N ; PAGES F ; MEJEAN A et al.** Adoptive immunotherapy for superficial bladder cancer with autologous macrophage activated killer cells. *J. Urol.,* 2002, vol. 168, 2373-2376
- **DE GRAMONT A ; GANGJI D ; LOUVET C et al.** Adoptive immunotherapy of ovarian carcinoma. *Gynecol. Oncol.,* 2002, vol. 86, 102-103

SEQUENCE LISTING

<110> Laboratoire Français du Fractionnement et des Biotechnologies

<120> Kit of parts for the treatment of hematopoietic tumor or autoimmune diseases

<130> Macrophages-EMABling

<160> 3

<170> PatentIn version 3.1

<210> 1

<211> 448

<212> PRT

<213> Artificial Sequence

<220>

<223> Synthetic Construct

<400> 1

```
Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Arg Gln Gly Leu Glu Trp Ile
        35                  40                  45

Gly Gly Ile Tyr Pro Gly Asn Gly Asp Thr Ser Tyr Asn Gln Lys Phe
    50                  55                  60

Lys Gly Lys Ala Thr Leu Thr Val Gly Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Tyr Asp Tyr Asn Tyr Ala Met Asp Tyr Trp Gly Gln Gly Thr
            100                 105                 110
```

```
Ser Val Thr Val Ser Ser Ala Ser Thr Lys Gly Pro Ser Val Phe Pro
        115             120             125

Leu Ala Pro Ser Ser Lys Ser Thr Ser Gly Gly Thr Ala Ala Leu Gly
        130             135             140

Cys Leu Val Lys Asp Tyr Phe Pro Glu Pro Val Thr Val Ser Trp Asn
145             150             155             160

Ser Gly Ala Leu Thr Ser Gly Val His Thr Phe Pro Ala Val Leu Gln
                165             170             175

Ser Ser Gly Leu Tyr Ser Leu Ser Ser Val Val Thr Val Pro Ser Ser
            180             185             190

Ser Leu Gly Thr Gln Thr Tyr Ile Cys Asn Val Asn His Lys Pro Ser
            195             200             205

Asn Thr Lys Val Asp Lys Lys Val Glu Pro Lys Ser Cys Asp Lys Thr
210             215             220

His Thr Cys Pro Pro Cys Pro Ala Pro Glu Leu Leu Gly Gly Pro Ser
225             230             235             240

Val Phe Leu Phe Pro Pro Lys Pro Lys Asp Thr Leu Met Ile Ser Arg
            245             250             255

Thr Pro Glu Val Thr Cys Val Val Val Asp Val Ser His Glu Asp Pro
            260             265             270

Glu Val Lys Phe Asn Trp Tyr Val Asp Gly Val Glu Val His Asn Ala
        275             280             285

Lys Thr Lys Pro Arg Glu Glu Gln Tyr Asn Ser Thr Tyr Arg Val Val
    290             295             300

Ser Val Leu Thr Val Leu His Gln Asp Trp Leu Asn Gly Lys Glu Tyr
305             310             315             320

Lys Cys Lys Val Ser Asn Lys Ala Leu Pro Ala Pro Ile Glu Lys Thr
            325             330             335

Ile Ser Lys Ala Lys Gly Gln Pro Arg Glu Pro Gln Val Tyr Thr Leu
        340             345             350

Pro Pro Ser Arg Asp Glu Leu Thr Lys Asn Gln Val Ser Leu Thr Cys
        355             360             365

Leu Val Lys Gly Phe Tyr Pro Ser Asp Ile Ala Val Glu Trp Glu Ser
    370             375             380
```

```
Asn Gly Gln Pro Glu Asn Asn Tyr Lys Thr Thr Pro Pro Val Leu Asp
385             390             395                 400

Ser Asp Gly Ser Phe Phe Leu Tyr Ser Lys Leu Thr Val Asp Lys Ser
            405             410                 415

Arg Trp Gln Gln Gly Asn Val Phe Ser Cys Ser Val Met His Glu Ala
        420             425             430

Leu His Asn His Tyr Thr Gln Lys Ser Leu Ser Leu Ser Pro Gly Lys
        435             440             445
```

<210> 2

<211> 213

<212> PRT

<213> Artificial Sequence


<220>

<223> Synthetic Construct

<400> 2

```
Gln Ile Val Leu Ser Gln Ser Pro Ala Ile Leu Ser Ala Ser Pro Gly
1               5               10              15

Glu Lys Val Thr Met Thr Cys Arg Ala Ser Ser Ser Val Ser Tyr Met
            20              25              30

His Trp Tyr Gln Gln Lys Pro Gly Ser Ser Pro Lys Pro Trp Ile Tyr
        35              40              45

Ala Thr Ser Asn Leu Ala Ser Gly Val Pro Ala Arg Phe Ser Gly Ser
    50              55              60

Gly Ser Gly Thr Ser Tyr Ser Phe Thr Ile Ser Arg Val Glu Ala Glu
65              70              75                  80

Asp Ala Ala Thr Tyr Tyr Cys Gln Gln Trp Thr Phe Asn Pro Pro Thr
            85              90              95

Phe Gly Gly Gly Thr Arg Leu Glu Ile Asn Arg Thr Val Ala Ala Pro
            100             105             110

Ser Val Phe Ile Phe Pro Pro Ser Asp Glu Gln Leu Lys Ser Gly Thr
        115             120             125

Ala Ser Val Val Cys Leu Leu Asn Asn Phe Tyr Pro Arg Glu Ala Lys
        130             135             140
```

```
Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
        210


<210>  3

<211>  213

<212>  PRT

<213>  Artificial Sequence


<220>

<223>  Synthetic Construct

<400>  3

Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Met
            20                  25                  30

His Trp Tyr Gln Gln Lys Pro Gly Ser Ser Pro Lys Pro Trp Ile Tyr
        35                  40                  45

Ala Thr Ser Asn Leu Ala Ser Gly Val Pro Ala Arg Phe Ser Gly Ser
    50                  55                  60

Gly Ser Gly Thr Ser Tyr Ser Phe Thr Ile Ser Arg Val Glu Ala Glu
65                  70                  75                  80

Asp Ala Ala Thr Tyr Tyr Cys Gln Gln Trp Thr Phe Asn Pro Pro Thr
                85                  90                  95

Phe Gly Gly Gly Thr Arg Leu Glu Ile Lys Arg Thr Val Ala Ala Pro
            100                 105                 110

Ser Val Phe Ile Phe Pro Pro Ser Asp Glu Gln Leu Lys Ser Gly Thr
            115                 120                 125
```

```
Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
                180                 185                 190

Cys Glu Val Thr His Gln Gly Leu Ser Ser Pro Val Thr Lys Ser Phe
        195                 200                 205

Asn Arg Gly Glu Cys
        210
```